# EUROPEAN PATENT APPLICATION

(11) **EP 4 595 960 A1**
(43) Date of publication of application: **06.08.2025**
(21) Application number: 23872569.1
(22) Date of filing: 28.09.2023
(51) Int. Cl.: A61K 31/353, A61K 9/10, A61K 31/196, A61K 31/56, A61K 31/57, A61K 31/7084, A61K 31/728, A61K 38/13, A61K 47/26, A61K 47/34, A61P 27/04, A61P 43/00

(54) **COMBINATION PREPARATION CONTAINING HETEROCYCLIDENE ACETAMIDE DERIVATIVE**

(30) Priority: 29.09.2022 JP 2022156062
(71) Applicant: Senju Pharmaceutical Co., Ltd., Osaka-shi, Osaka 541-0048 (JP); MOCHIDA PHARMACEUTICAL CO., LTD., Tokyo 160-8515 (JP)
(72) Inventor: YAMASHITA, Yuki, Osaka-shi, Osaka 541-0048 (JP)
(74) Representative: Engelhard, Markus
(86) International application number: PCT/JP2023/035548
(87) International publication number: WO 2024/071349

(57) **Abstract**

The present disclosure provides a suspension-type ophthalmic solution having excellent re-dispersibility. More specifically, the present disclosure provides a water-based suspension preparation containing a first component and a second component, in which the first component is (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof, and the second component comprises at least one component selected from the group consisting of diquafosol, ciclosporin, hyaluronic acid, loteprednol etabonate, bromfenac, fluorometholone and pharmaceutically acceptable salts or solvates thereof.

## Description

### [Technical Field]

The present disclosure relates to the fields of pharmaceuticals, health care, biology, biotechnology, or the like. In particular, the present disclosure relates to a technique for improving redispersibility of a suspension liquid containing a heterocyclidene acetamide derivative; and a formulation technique based thereon.

### [Background Art]

The number of dry eye patients in Japan is estimated to be at least about 8 million, or about 22 million including potential patients who use over-the-counter ophthalmic solutions without going to the hospital. It is said that there are 1 billion or more dry eye patients worldwide. It has been widely known that in modern society, use of televisions, computers, mobile terminals, etc. causes people to stare at screens more frequently to thereby reduce eye blink frequency, and use of air conditioners, etc. dries out the air, resulting in increased evaporation of tear fluid and thus a dry eye. Refractive surgery and use of contact lenses also result in a dry eye. Symptoms associated with the dry eye include, for example, ocular discomfort, dryness, burning, and irritation of an ocular surface.

When the dry eye occurs, the above symptoms appear as subjective symptoms, and their treatment requires regular eye drops over a long period of time. For this reason, most of the dry eye therapeutic drugs currently on the market are generally frequent-dosing types. For example, typically, a DIQUAS (registered trademark) ophthalmic solution is required to be applied 6 times a day, a HYALEIN (registered trademark) ophthalmic solution is required to be applied 5 to 6 times a day, and a MUCOSTA (registered trademark) ophthalmic solution is required to be applied 4 times a day.

A composition including (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof, or use thereof for treating a dry eye has been disclosed (PTL 1).

### [Citation List]

### [Patent Literature]

[PTL 1] International Publication No. WO 2021/066144

### [Summary of Invention]

### [Technical Problem]

The present disclosure provides an aqueous suspension liquid formulation with excellent redispersibility, the formation including, as a first component, (E)-2-(7-trifluoromethylchroman-4-ylidene)-N-(7-hydroxy-5,6,7,8-tetrahydronaphthalen-1-yl)acetamide, which is one of heterocyclidene acetamide derivatives, or a pharmaceutically acceptable salt or solvate thereof; and, as a second component, at least one selected from the group consisting of diquafosol, cyclosporine, hyaluronic acid, loteprednol etabonate, bromfenac, fluorometholone, and a pharmaceutically acceptable salt or solvate thereof.

Therefore, the present disclosure provides the following items.

### (Item 1)

An aqueous suspension liquid formulation including:
a first component; and
a second component,
the first component being (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof, and
the second component being at least one selected from the group consisting of diquafosol, cyclosporine, hyaluronic acid, loteprednol etabonate, bromfenac, fluorometholone, and a pharmaceutically acceptable salt or solvate thereof.

### (Item 2)

The aqueous suspension liquid formulation according to the above item, further including a nonionic surfactant.

### (Item 3)

The aqueous suspension liquid formulation according to any one of the above items, in which the nonionic surfactant is at least one selected from the group consisting of tyloxapol, polysorbate, polyethylene glycol monostearate, and a polyoxyethylene hydrogenated castor oil.

### (Item 4)

The aqueous suspension liquid formulation according to any one of the above items, in which the nonionic surfactant is at least one selected from the group consisting of tyloxapol, polysorbate 80, polyethylene glycol monostearate 40, and a polyoxyethylene hydrogenated castor oil 60.

### (Item 5)

The aqueous suspension liquid formulation according to any one of the above items, in which the nonionic surfactant is tyloxapol.

### (Item 6)

The aqueous suspension liquid formulation according to any one of the above items, in which the nonionic surfactant has a concentration of about 0.0001 w/v% to about 1 w/v% in the aqueous suspension liquid formulation.

### (Item 7)

The aqueous suspension liquid formulation according to any one of the above items, in which the first component has a concentration of about 0.01 w/v% to about 5 w/v% in the aqueous suspension liquid formulation.

### (Item 8)

The aqueous suspension liquid formulation according to any one of the above items, in which the first component has a concentration of about 0.3 w/v% to about 1 w/v% in the aqueous suspension liquid formulation.

### (Item 9)

The aqueous suspension liquid formulation according to any one of the above items, in which the second component has a concentration of about 0.005 w/v% to about 10 w/v% in the aqueous suspension liquid formulation.

### (Item 10)

The aqueous suspension liquid formulation according to any one of the above items, in which the diquafosol or a pharmaceutically acceptable salt or solvate thereof has a concentration of about 0.3 w/v% to about 10 w/v% in the aqueous suspension liquid formulation,
the cyclosporine or a pharmaceutically acceptable salt or solvate thereof has a concentration of about 0.005 w/v% to about 0.5 w/v% in the aqueous suspension liquid formulation,
the hyaluronic acid or a pharmaceutically acceptable salt or solvate thereof has a concentration of about 0.01 w/v% to about 1 w/v% in the aqueous suspension liquid formulation,
the loteprednol etabonate or a pharmaceutically acceptable salt or solvate thereof has a concentration of about 0.025 w/v% to about 2.5 w/v% in the aqueous suspension liquid formulation,
the bromfenac or a pharmaceutically acceptable salt or solvate thereof has a concentration of about 0.01 w/v% to about 1 w/v% in the aqueous suspension liquid formulation, and
the fluorometholone or a pharmaceutically acceptable salt or solvate thereof has a concentration of about 0.01 w/v% to about 1 w/v% in the aqueous suspension liquid formulation.

### (Item 11)

The aqueous suspension liquid formulation according to any one of the above items, in which the first component is (E)-2-(7-trifluoromethylchroman-4-ylidene)-N-((7R)-7-hydroxy-5,6,7,8-tetrahydronaphthalen-1-yl)acetamide or a pharmaceutically acceptable salt or solvate thereof.

### (Item 12)

The aqueous suspension liquid formulation according to any one of the above items, further including boric acid.

### (Item 13)

The aqueous suspension liquid formulation according to any one of the above items, in which the aqueous suspension liquid formulation has a pH of about 4 to about 8.

### (Item 14)

The aqueous suspension liquid formulation according to any one of the above items, in which the (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof has improved redispersibility in the aqueous suspension liquid formulation.

### (Item 15)

The aqueous suspension liquid formulation according to any one of the above items, in which the improved redispersibility is a property of redispersing suspended particles in about 30 inversions or less as evaluated by an inversion operation.

### (Item A1)

An aqueous suspension liquid formulation including:
a first component;
a second component; and
a nonionic surfactant,
the first component being (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof,
the second component being at least one selected from the group consisting of diquafosol, cyclosporine, hyaluronic acid, loteprednol etabonate, bromfenac, fluorometholone, and a pharmaceutically acceptable salt or solvate thereof, and
the nonionic surfactant being at least one selected from the group consisting of tyloxapol, polysorbate, polyethylene glycol monostearate, and a polyoxyethylene hydrogenated castor oil.

### (Item A2)

An aqueous suspension liquid formulation including:
a first component;
a second component; and
a nonionic surfactant,
the first component being (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof,
the second component being at least one selected from the group consisting of diquafosol, cyclosporine, hyaluronic acid, loteprednol etabonate, bromfenac, fluorometholone, and a pharmaceutically acceptable salt or solvate thereof, and
the nonionic surfactant being at least one selected from the group consisting of tyloxapol, polysorbate 80, polyethylene glycol monostearate 40, and a polyoxyethylene hydrogenated castor oil 60.

### (Item A3)

An aqueous suspension liquid formulation including:
a first component;
a second component; and
a nonionic surfactant,
the first component being (E)-2-(7-trifluoromethylchroman-4-ylidene)-N-((7R)-7-hydroxy-5,6,7,8-tetrahydronaphthalen-1-yl)acetamide or a pharmaceutically acceptable salt or solvate thereof,
the second component being at least one selected from the group consisting of diquafosol, cyclosporine, hyaluronic acid, loteprednol etabonate, bromfenac, fluorometholone, and a pharmaceutically acceptable salt or solvate thereof,
the nonionic surfactant being at least one selected from the group consisting of tyloxapol, polysorbate 80, polyethylene glycol monostearate 40, and a polyoxyethylene hydrogenated castor oil 60,
the first component having a concentration of about 0.01 w/v% to about 5 w/v% in the aqueous suspension liquid formulation,
the second component having a concentration of about 0.005 w/v% to about 10 w/v% in the aqueous suspension liquid formulation, and
the nonionic surfactant having a concentration of about 0.0001 w/v% to about 1 w/v% in the aqueous suspension liquid formulation.

### (Item A4)

An aqueous suspension liquid formulation including:
a first component;
a second component; and
a nonionic surfactant,
the first component being (E)-2-(7-trifluoromethylchroman-4-ylidene)-N-((7R)-7-hydroxy-5,6,7,8-tetrahydronaphthalen-1-yl)acetamide or a pharmaceutically acceptable salt or solvate thereof,
the second component being at least one selected from the group consisting of diquafosol, cyclosporine, hyaluronic acid, loteprednol etabonate, bromfenac, fluorometholone, and a pharmaceutically acceptable salt or solvate thereof,
the nonionic surfactant being at least one selected from the group consisting of tyloxapol, polysorbate 80, polyethylene glycol monostearate 40, and a polyoxyethylene hydrogenated castor oil 60,
the first component having a concentration of about 0.01 w/v% to about 5 w/v% in the aqueous suspension liquid formulation,
the diquafosol or a pharmaceutically acceptable salt or solvate thereof having a concentration of about 0.3 w/v% to about 10 w/v% in the aqueous suspension liquid formulation,
the cyclosporine or a pharmaceutically acceptable salt or solvate thereof having a concentration of about 0.005 w/v% to about 0.5 w/v% in the aqueous suspension liquid formulation,
the hyaluronic acid or a pharmaceutically acceptable salt or solvate thereof having a concentration of about 0.01 w/v% to about 1 w/v% in the aqueous suspension liquid formulation,
the loteprednol etabonate or a pharmaceutically acceptable salt or solvate thereof having a concentration of about 0.025 w/v% to about 2.5 w/v% in the aqueous suspension liquid formulation,
the bromfenac or a pharmaceutically acceptable salt or solvate thereof having a concentration of about 0.01 w/v% to about 1 w/v% in the aqueous suspension liquid formulation,
the fluorometholone or a pharmaceutically acceptable salt or solvate thereof having a concentration of about 0.01 w/v% to about 1 w/v% in the aqueous suspension liquid formulation, and
the nonionic surfactant having a concentration of about 0.0001 w/v% to about 1 w/v% in the aqueous suspension liquid formulation.

### (Item A5)

An aqueous suspension liquid formulation including:
a first component;
a second component; and
a nonionic surfactant,
the first component being (E)-2-(7-trifluoromethylchroman-4-ylidene)-N-((7R)-7-hydroxy-5,6,7,8-tetrahydronaphthalen-1-yl)acetamide or a pharmaceutically acceptable salt or solvate thereof,
the second component being at least one selected from the group consisting of diquafosol, cyclosporine, hyaluronic acid, loteprednol etabonate, bromfenac, fluorometholone, and a pharmaceutically acceptable salt or solvate thereof,
the nonionic surfactant being at least one selected from the group consisting of tyloxapol, polysorbate 80, polyethylene glycol monostearate 40, and a polyoxyethylene hydrogenated castor oil 60,
the first component having a concentration of about 0.3 w/v% to about 1 w/v% in the aqueous suspension liquid formulation,
the diquafosol or a pharmaceutically acceptable salt or solvate thereof having a concentration of about 3 w/v% in the aqueous suspension liquid formulation,
the cyclosporine or a pharmaceutically acceptable salt or solvate thereof having a concentration of about 0.05 w/v% in the aqueous suspension liquid formulation,
the hyaluronic acid or a pharmaceutically acceptable salt or solvate thereof having a concentration of about 0.1 w/v% in the aqueous suspension liquid formulation,
the loteprednol etabonate or a pharmaceutically acceptable salt or solvate thereof having a concentration of about 0.25 w/v% in the aqueous suspension liquid formulation, and
the nonionic surfactant having a concentration of about 0.01 w/v% to about 0.05 w/v% in the aqueous suspension liquid formulation.

### (Item A6)

An aqueous suspension liquid formulation including:
a first component;
a second component;
a nonionic surfactant; and
boric acid,
the first component being (E)-2-(7-trifluoromethylchroman-4-ylidene)-N-((7R)-7-hydroxy-5,6,7,8-tetrahydronaphthalen-1-yl)acetamide or a pharmaceutically acceptable salt or solvate thereof,
the second component being at least one selected from the group consisting of diquafosol, cyclosporine, hyaluronic acid, loteprednol etabonate, bromfenac, fluorometholone, and a pharmaceutically acceptable salt or solvate thereof,
the nonionic surfactant being at least one selected from the group consisting of tyloxapol, polysorbate 80, polyethylene glycol monostearate 40, and a polyoxyethylene hydrogenated castor oil 60,
the first component having a concentration of about 0.3 w/v% in the aqueous suspension liquid formulation,
the diquafosol or a pharmaceutically acceptable salt or solvate thereof having a concentration of about 3 w/v% in the aqueous suspension liquid formulation,
the cyclosporine or a pharmaceutically acceptable salt or solvate thereof having a concentration of about 0.05 w/v% in the aqueous suspension liquid formulation,
the hyaluronic acid or a pharmaceutically acceptable salt or solvate thereof having a concentration of about 0.1 w/v% in the aqueous suspension liquid formulation,
the loteprednol etabonate or a pharmaceutically acceptable salt or solvate thereof having a concentration of about 0.25 w/v% in the aqueous suspension liquid formulation,
the bromfenac or a pharmaceutically acceptable salt or solvate thereof having a concentration of about 0.1 w/v% in the aqueous suspension liquid formulation,
the fluorometholone or a pharmaceutically acceptable salt or solvate thereof having a concentration of about 0.1 w/v% in the aqueous suspension liquid formulation,
the nonionic surfactant having a concentration of about 0.01 w/v% to about 0.05 w/v% in the aqueous suspension liquid formulation,
the aqueous suspension liquid formulation having a pH of about 4 to about 8, and
the aqueous suspension liquid formulation including suspended particles redispersed in about 30 inversions or less as evaluated for redispersibility of the aqueous suspension liquid formulation by an inversion operation.

### (Item B1)

A method for producing an aqueous suspension liquid formulation, the method including:
mixing a first component, a second component, and a nonionic surfactant to prepare the aqueous suspension liquid formulation,
the first component being (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof,
the second component being at least one selected from the group consisting of diquafosol, cyclosporine, hyaluronic acid, loteprednol etabonate, bromfenac, fluorometholone, and a pharmaceutically acceptable salt or solvate thereof, and
the nonionic surfactant being at least one selected from the group consisting of tyloxapol, polysorbate, polyethylene glycol monostearate, and a polyoxyethylene hydrogenated castor oil.

### (Item C1)

A method for improving redispersibility of an aqueous suspension liquid formulation, the method including:
mixing a first component, a second component, and a nonionic surfactant to prepare the aqueous suspension liquid formulation,
the first component being (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof,
the second component being at least one selected from the group consisting of diquafosol, cyclosporine, hyaluronic acid, loteprednol etabonate, bromfenac, fluorometholone, and a pharmaceutically acceptable salt or solvate thereof, and
the nonionic surfactant being at least one selected from the group consisting of tyloxapol, polysorbate, polyethylene glycol monostearate, and a polyoxyethylene hydrogenated castor oil.

### [Advantageous Effects of Invention]

The present disclosure can provide an aqueous suspension liquid formulation with excellent redispersibility.

By providing the above features, the present disclosure suppresses a phenomenon that suspended particles are not uniformly dispersed, enables stable administration of a required amount of an active ingredient and realizes of a stable and sufficient exertion of pharmacological effect. This contributes to improvement of patient adherence and convenience.

### [Brief Description of Drawings]

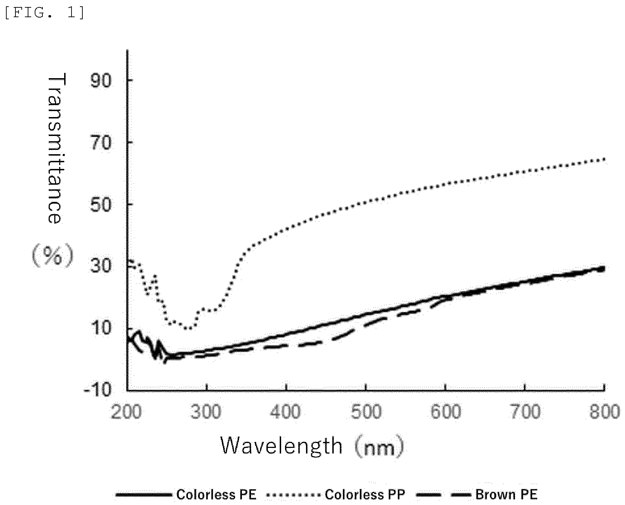

[FIG. 1] FIG. 1 shows transmittance at various wavelengths for a body of a bottle of each of containers used in Examples.

### [Description of Embodiments]

The present disclosure will be described. It should be understood that throughout this specification, singular expressions also include the concept of their plural forms, unless otherwise noted. Thus, it should be understood that singular articles (e.g., "a", "an", "the", etc. in English) also include the concept of their plural forms, unless otherwise noted. It should also be understood that terms used herein are to be used in the sense normally used in the art, unless otherwise noted. Therefore, unless otherwise defined, all technical and scientific terms used herein have the same meaning as commonly understood by those skilled in the art to which the present disclosure pertains. In the event of a conflict, this specification (including definitions) shall prevail.

### (Definitions)

As used herein, the term "about" means ± 10% of the numeral value that follows, unless otherwise noted.

As used herein, the term "or" is used when "at least one or more" of matters enumerated in a text can be employed. The same is true of "alternatively". When the phrase "within a range" of "two values" is specified herein, the range includes the two values themselves.

As used herein, the phrase "aqueous suspension liquid formulation" is used with the same meaning as the term commonly used in the art and refers to a liquid formulation that contains water in at least a portion thereof, in which components to be mixed are in suspension and in which solid particles are present in the liquid. Since (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof serving as a first component has extremely less soluble in water, the (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof becomes suspended particles, but partially dissolved in an aqueous suspension liquid formulation of the present disclosure. In addition to the first component, a second component may also be suspended particles.

As used herein, the phrase "pharmaceutically acceptable salt" means a relatively non-toxic, inorganic or organic acid addition salt or inorganic or organic base addition salt of a compound of the present disclosure.

As used herein, the term "solvate" means one aggregate formed by interaction of a compound of the present disclosure or a pharmaceutically acceptable salt thereof with any solvent, and includes, for example, a solvate with an organic solvent (e.g., an alcoholate (ethanolate, etc.)) and a hydrate. When the hydrate is formed, the compound of the present disclosure or the pharmaceutically acceptable salt thereof may be coordinated with any number of water molecules. The hydrate may include a monohydrate and a dihydrate and the like.

As used herein, the term "redispersibility" refers to ease of uniform dispersion of particles (also referred to as "suspended particles" in the case of a suspension liquid) that have settled in a solvent of a liquid containing the particles, such as a suspension liquid, throughout the liquid again after the liquid is stored for a certain period of time. As used herein, the term "redispersibility" is evaluated by testing with a shaking or inversion operation.

As used herein, the phrase "shaking operation" refers to an action of holding a container filled with the "aqueous suspension liquid formulation" in the hand and shaking it up and down. For the "shaking operation", the container is shaken down 10 to 15 cm vertically and then shaken up to its original position, which is defined as one shake. Five shakes are determined as one set and the contained is shaken at a rate of 1.1 seconds/set. A test for the redispersibility with the "shaking operation" is performed on 3 to 6 samples per formulation, and an average number of sets is calculated from the number of sets required for each sample to redisperse, which is then converted to the number of shakes.

As used herein, the phrase "inversion operation" refers to an action of holding a container filled with the "aqueous suspension liquid formulation" in the hand and turning it up and down. A test for the redispersibility with the "inversion operation" is performed on 3 to 6 samples per formulation. The container is turned 180° vertically and then turned 180° again to stand upright at a rate of 1 second/inversion, which is defined as one inversion.

As used herein, the phrase "improved redispersibility" means that particles that have settled in a liquid containing the particles, such as a suspension liquid, are easier to disperse again, and if the number of actions of the above "shaking operation" or "inversion operation" is reduced in comparison for any formulations, the redispersibility can be said to be improved.

As used herein, the phrase "average particle size of suspended particles" refers to a median diameter (D₅₀) of particle diameters of particles of (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof and is measured by a laser diffraction particle size distribution analyzer.

As used herein, the phrase "nonionic surfactant" is also referred to as a non-ion surfactant, and refers to a surfactant of which hydrophilic group portion is nonionic. Those skilled in the art can easily determine whether a compound is a nonionic surfactant or not by checking that the compound does not ionize (show ionicity) when dissolved in water. Examples of the nonionic surfactant include tyloxapol, polyoxyl stearates (polyethylene glycol monostearate, polyethylene glycol monostearate 40 (MYS-40), polyethylene glycol monostearate 400, etc.), polyoxyethylene sorbitan fatty acid esters (polyoxyethylene sorbitan monooleate (polysorbate 80), polyoxyethylene sorbitan monolaurate, polyoxyethylene sorbitan monopalmitate, polyoxyethylene sorbitan monostearate, polyoxyethylene sorbitan tristearate, etc.), and polyoxyethylene hydrogenated castor oils (polyoxyethylene hydrogenated castor oil 10, polyoxyethylene hydrogenated castor oil 40, polyoxyethylene hydrogenated castor oil 50, polyoxyethylene hydrogenated castor oil 60 (HCO-60), etc.).

### (Preferred Embodiment)

Preferred embodiments of the present disclosure will be described. Embodiments provided below are given for a better understanding of the present disclosure and the scope of the present disclosure should not be limited to the following description. Therefore, it is clear that those skilled in the art may make modifications as appropriate within the scope of the present disclosure, taking into account the description herein. The following embodiments of the present disclosure may also be used alone or in combination.

### (Aqueous suspension liquid formulation)

The present disclosure may provide an aqueous suspension liquid formulation including a first component and a second component, the first component being (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof, and the second component being at least one selected from the group consisting of other active components and a pharmaceutically acceptable salt or solvate thereof.

The present disclosure may provide an aqueous suspension liquid formulation including a first component and a second component, the first component being (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof, and the second component being at least one selected from the group consisting of diquafosol, cyclosporine, hyaluronic acid, loteprednol etabonate, bromfenac, fluorometholone, and a pharmaceutically acceptable salt or solvate thereof.

The present disclosure may provide an aqueous suspension liquid formulation including a first component, a second component, and a nonionic surfactant, the first component being (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof, and the second component being at least one selected from the group consisting of diquafosol, cyclosporine, hyaluronic acid, loteprednol etabonate, bromfenac, fluorometholone, and a pharmaceutically acceptable salt or solvate thereof, and the nonionic surfactant being at least one selected from the group consisting of tyloxapol, polysorbate, polyethylene glycol monostearate, and a polyoxyethylene hydrogenated castor oil.

The present disclosure may provide an aqueous suspension liquid formulation including a first component, a second component, and a nonionic surfactant, the first component being (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof, and the second component being at least one selected from the group consisting of diquafosol, cyclosporine, hyaluronic acid, loteprednol etabonate, bromfenac, fluorometholone, and a pharmaceutically acceptable salt or solvate thereof, and the nonionic surfactant being at least one selected from the group consisting of tyloxapol, polysorbate 80, polyethylene glycol monostearate 40, and a polyoxyethylene hydrogenated castor oil 60.

The present disclosure may provide an aqueous suspension liquid formulation including a first component, a second component, a nonionic surfactant, and boric acid, the first component being (E)-2-(7-trifluoromethylchroman-4-ylidene)-N-((7R)-7-hydroxy-5,6,7,8-tetrahydronaphthalen-1-yl)acetamide or a pharmaceutically acceptable salt or solvate thereof, and the second component being at least one selected from the group consisting of diquafosol, cyclosporine, hyaluronic acid, loteprednol etabonate, bromfenac, fluorometholone, and a pharmaceutically acceptable salt or solvate thereof, and the nonionic surfactant being at least one selected from the group consisting of tyloxapol, polysorbate 80, polyethylene glycol monostearate 40, and a polyoxyethylene hydrogenated castor oil 60.

Another aspect of the present disclosure may provide an aqueous suspension liquid formulation including a first component and a second component, the first component being (E)-2-(7-trifluoromethylchroman-4-ylidene)-N-((7R)-7-hydroxy-5,6,7,8-tetrahydronaphthalen-1-yl)acetamide or a pharmaceutically acceptable salt or solvate thereof, and the second component being at least one selected from the group consisting of rebamipide, olodaterol, delgocitinib, and a pharmaceutically acceptable salt or solvate thereof; or alternatively, an aqueous suspension liquid formulation including a first component, a second component, a nonionic surfactant, and boric acid, the first component being (E)-2-(7-trifluoromethylchroman-4-ylidene)-N-((7R)-7-hydroxy-5,6,7,8-tetrahydronaphthalen-1-yl)acetamide or a pharmaceutically acceptable salt or solvate thereof, and the second component being at least one selected from the group consisting of rebamipide, olodaterol, delgocitinib, and a pharmaceutically acceptable salt or solvate thereof; and the nonionic surfactant being at least one selected from the group consisting of tyloxapol, polysorbate 80, polyethylene glycol monostearate 40, and a polyoxyethylene hydrogenated castor oil 60.

One aspect of the present disclosure may provide a method for producing an aqueous suspension liquid formulation, the method including mixing a first component and a second component to prepare the aqueous suspension liquid formulation, the first component being (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof, and the second component being at least one selected from the group consisting of diquafosol, cyclosporine, hyaluronic acid, loteprednol etabonate, bromfenac, fluorometholone, and a pharmaceutically acceptable salt or solvate thereof.

Another aspect may also provide a method for producing an aqueous suspension liquid formulation, the method including mixing a first component, a second component, and a nonionic surfactant to prepare the aqueous suspension liquid formulation, the first component being (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof, and the second component being at least one selected from the group consisting of diquafosol, cyclosporine, hyaluronic acid, loteprednol etabonate, bromfenac, fluorometholone, and a pharmaceutically acceptable salt or solvate thereof, and the nonionic surfactant being at least one selected from the group consisting of tyloxapol, polysorbate, polyethylene glycol monostearate, and a polyoxyethylene hydrogenated castor oil.

Another aspect may also provide a method for producing an aqueous suspension liquid formulation, the method including mixing a first component, a second component, and a nonionic surfactant to prepare the aqueous suspension liquid formulation, the first component being (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof, and the second component being at least one selected from the group consisting of diquafosol, cyclosporine, hyaluronic acid, loteprednol etabonate, bromfenac, fluorometholone, and a pharmaceutically acceptable salt or solvate thereof, and the nonionic surfactant being at least one selected from the group consisting of tyloxapol, polysorbate 80, polyethylene glycol monostearate 40, and a polyoxyethylene hydrogenated castor oil 60.

Another aspect may also provide a method for producing an aqueous suspension liquid formulation, the method including mixing a first component, a second component, a nonionic surfactant, and boric acid to prepare the aqueous suspension liquid formulation, the first component being (E)-2-(7-trifluoromethylchroman-4-ylidene)-N-((7R)-7-hydroxy-5,6,7,8-tetrahydronaphthalen-1-yl)acetamide or a pharmaceutically acceptable salt or solvate thereof, and the second component being at least one selected from the group consisting of diquafosol, cyclosporine, hyaluronic acid, loteprednol etabonate, bromfenac, fluorometholone, and a pharmaceutically acceptable salt or solvate thereof, and the nonionic surfactant being at least one selected from the group consisting of tyloxapol, polysorbate 80, polyethylene glycol monostearate 40, and a polyoxyethylene hydrogenated castor oil 60.

Another aspect of the present disclosure may provide a method for improving redispersibility of an aqueous suspension liquid formulation, the method including mixing a first component and a second component to prepare the aqueous suspension liquid formulation, the first component being (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof, and the second component being at least one selected from the group consisting of diquafosol, cyclosporine, hyaluronic acid, loteprednol etabonate, bromfenac, fluorometholone, and a pharmaceutically acceptable salt or solvate thereof.

Another aspect may also provide a method for improving redispersibility of an aqueous suspension liquid formulation, the method including mixing a first component, a second component, and a nonionic surfactant to prepare the aqueous suspension liquid formulation, the first component being (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof, and the second component being at least one selected from the group consisting of diquafosol, cyclosporine, hyaluronic acid, loteprednol etabonate, bromfenac, fluorometholone, and a pharmaceutically acceptable salt or solvate thereof, and the nonionic surfactant being at least one selected from the group consisting of tyloxapol, polysorbate, polyethylene glycol monostearate, and a polyoxyethylene hydrogenated castor oil.

Another aspect may also provide a method for improving redispersibility of an aqueous suspension liquid formulation, the method including mixing a first component, a second component, and a nonionic surfactant to prepare the aqueous suspension liquid formulation, the first component being (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof, and the second component being at least one selected from the group consisting of diquafosol, cyclosporine, hyaluronic acid, loteprednol etabonate, bromfenac, fluorometholone, and a pharmaceutically acceptable salt or solvate thereof, and the nonionic surfactant being at least one selected from the group consisting of tyloxapol, polysorbate 80, polyethylene glycol monostearate 40, and a polyoxyethylene hydrogenated castor oil 60.

Another aspect may also provide a method for improving redispersibility of an aqueous suspension liquid formulation, the method including mixing a first component, a second component, a nonionic surfactant, and boric acid to prepare the aqueous suspension liquid formulation, the first component being (E)-2-(7-trifluoromethylchroman-4-ylidene)-N-((7R)-7-hydroxy-5,6,7,8-tetrahydronaphthalen-1-yl)acetamide or a pharmaceutically acceptable salt or solvate thereof, and the second component being at least one selected from the group consisting of diquafosol, cyclosporine, hyaluronic acid, loteprednol etabonate, bromfenac, fluorometholone, and a pharmaceutically acceptable salt or solvate thereof, and the nonionic surfactant being at least one selected from the group consisting of tyloxapol, polysorbate 80, polyethylene glycol monostearate 40, and a polyoxyethylene hydrogenated castor oil 60.

In the present disclosure, (E)-2-(7-trifluoromethylchroman-4-ylidene)-N-(7-hydroxy-5,6,7,8-tetrahydronaphthalen-1-yl)acetamide includes a R isomer thereof (CAS. No. 920332-28-1), a S isomer thereof (CAS. No. 920332-29-2), or a racemate thereof (CAS. No. 920332-27-0), more preferably is the R isomer thereof ((E)-2-(7-trifluoromethylchroman-4-ylidene)-N-((7R)-7-hydroxy-5,6,7,8-tetrahydronaphthalen-1-yl)acetamide (herein also referred to as Compound (1)). The compound (1) that may be used in the present disclosure may be provided in any form, for example, it may be a sterilized compound (1) or a non-sterilized compound (1). Examples of a sterilization method include dry heat sterilization, high-pressure steam sterilization, filtration sterilization, electron beam sterilization, a gamma ray, ethylene oxide gas sterilization, or hydrogen peroxide gas sterilization, with the dry heat sterilization being suitably used. A temperature at which the dry heat sterilization is carried out is not particularly limited and is usually about 100 to about 175 °C, preferably about 100 to about 170 °C, and more preferably about 150 to about 170 °C.

In the present disclosure, diquafosol is a compound incorporated as an active ingredient in its salt form (IUPAC: Tetrasodium P¹,P⁴-bis(5'-uridul)tetraphosphate/CAS. No. 211427-08-6) in a DIQUAS ophthalmic solution. A commercially available compound, a compound produced by a method described in a publication, or a pharmaceutically acceptable salt or solvate thereof can be used as the diquafosol or a pharmaceutically acceptable salt or solvate thereof.

In the present disclosure, cyclosporine is a compound incorporated as an active ingredient (IUPAC: cyclo{-[(2S,3R,4R,6E)-3-Hydroxy-4-methyl-2-methylaminooct-6-enoyl]-L-2-aminobutanoyl-N-methylglycyl-N-methyl-L-leucyl-L-valyl-N-methyl-L-leucyl-L-alanyl-D-alanyl-N-methyl-L-leucyl-N-methyl-L-leucyl-N-methyl-L-valyl-}/CAS.No.59865-13-3) in a RESTASIS ophthalmic solution. A commercially available compound, a compound produced by a method described in a publication, or a pharmaceutically acceptable salt or solvate thereof can be used as the cyclosporine or a pharmaceutically acceptable salt or solvate thereof.

In the present disclosure, hyaluronic acid is a compound incorporated as an active ingredient in its salt form (IUPAC: [→3)-2-Acetamido-2-deoxy-β-D-glucopyranosyl-(1→4)-β-D-glucopyranosyluronic acid-(1→]ₙ/CAS.9067-32-7) in a HYALEIN ophthalmic solution. A weight average molecular weight of the hyaluronic acid or a pharmaceutically acceptable salt or solvate thereof is usually from 500,000 to 1,200,000 and preferably from 600,000 to 1,200,000. A commercially available compound, a compound produced by a method described in a publication, or a pharmaceutically acceptable salt or solvate thereof can be used as the hyaluronic acid or a pharmaceutically acceptable salt or solvate thereof.

In the present disclosure, loteprednol etabonate is a compound incorporated as an active ingredient (IUPAC: Chloromethyl 17-ethoxycarbonyloxy-11-hydroxy-10,13-dimethyl-3-oxo-7,8,9,11,12,14,15,16-octahydro-6H-cyclopenta[a]phenanthrene-17-carboxylate/CAS No. 82034-46-6) in a EYSUVIS ophthalmic solution. A commercially available compound, a compound produced by a method described in a publication, or a pharmaceutically acceptable salt or solvate thereof can be used as the loteprednol etabonate or a pharmaceutically acceptable salt or solvate thereof.

In the present disclosure, bromfenac is a compound incorporated as an active ingredient in its salt form (Sodium 2 -[2-amino-3-(4-bromobenzoyl)phenyl acetate sesquihydrate/CAS. No. 120638-55-3) in a BRONUCK ophthalmic solution. A commercially available compound, a compound produced by a method described in a publication, or a pharmaceutically acceptable salt or solvate thereof can be used as the bromfenac or a pharmaceutically acceptable salt or solvate thereof.

In the present disclosure, fluorometholone is a compound incorporated as an active ingredient (IUPAC: 9-Fluoro-11β,17-dihydroxy-6α-methylpregna-1,4-diene-3,20-dione/CAS. No. 426-13 -1) in a FLUMETHOLON ophthalmic solution. A commercially available compound, a compound produced by a method described in a publication, or a pharmaceutically acceptable salt or solvate thereof can be used as the fluorometholone or a pharmaceutically acceptable salt or solvate thereof.

In the present disclosure, rebamipide is a compound incorporated as an active ingredient (IUPAC: (2RS)-2-(4-Chlorobenzoylamino)-3-(2-oxo-1,2-dihydroquinolin-4-yl)propanoic acid/CAS. No. 90098-04-7) in a MUCOSTA ophthalmic solution. A commercially available compound, a compound produced by a method described in a publication, or a pharmaceutically acceptable salt or solvate thereof can be used as the rebamipide or a pharmaceutically acceptable salt or solvate thereof.

In the present disclosure, olodaterol is a compound incorporated as an active ingredient in its salt form (6-Hydroxy-8-((1R)-1-hydroxy-2-{[1-(4-methoxyphenyl)-2-methylpropane-2-yl]amino}ethyl)-2H-1,4-benzooxazine-3(4H)-one·hydrochloride/CAS. No. 869477-96-3) in a SPIOLTO RESPIMAT 28 inhalant. A commercially available compound, a compound produced by a method described in a publication, or a pharmaceutically acceptable salt or solvate thereof can be used as the olodaterol or a pharmaceutically acceptable salt or solvate thereof.

In the present disclosure, dercositinib is a compound known as a Janus kinase (JAK) inhibitor (IUPAC: 3-[(3S,4R)-3-Methyl-6-(7H-pyrrolo[2,3-d]pyrimidin-4-yl)-1,6-diazaspiro[3.4]octan-1-yl]-3-oxopropanenitrile/CAS No. 869477-96-3). A commercially available compound, a compound produced by a method described in a publication, or a pharmaceutically acceptable salt or solvate thereof can be used as the dercositinib or a pharmaceutically acceptable salt or solvate thereof.

The pharmaceutically acceptable salt of the first component and the second component of the present disclosure each independently is not particularly limited as long as it is a pharmaceutically acceptable salt. Specifical examples thereof include mineral acids such as hydrochloric acid, hydrobromic acid, hydroiodic acid, sulfuric acid, nitric acid, phosphoric acid, etc.; organic carboxylic acids, for example, aliphatic monocarboxylic acids such as formic acid, acetic acid, propionic acid, butyric acid, valeric acid, enanthic acid, capric acid, myristic acid, palmitic acid, stearic acid, lactic acid, sorbic acid, mandelic acid, etc., aromatic monocarboxylic acids such as benzoic acid, salicylic acid, etc., aliphatic dicarboxylic acids such as oxalic acid, malonic acid, succinic acid, fumaric acid, maleic acid, malic acid, tartaric acid, etc. and aliphatic tricarboxylic acids such as citric acid, etc.; acid addition salts with organic sulfonic acids, for example, aliphatic sulfonic acids such as methanesulfonic acid, ethanesulfonic acid, 2-hydroxyethanesulfonic acid, etc. and aromatic sulfonic acids such as benzene sulfonic acid, p-toluenesulfonic acid, etc.; and inorganic base addition salts with metals, for example, alkali metals or alkaline earth metals such as sodium, potassium, magnesium, calcium, etc. and organic base addition salts such as methylamine, ethylamine, ethanolamine, pyridine, lysine, arginine, ornithine, etc.

These salts can be obtained by a routine procedure, e.g., by mixing equivalent amounts of the compound of the present disclosure with a solution containing a desired acid or base, and then filtering out a desired salt or collecting the desired salt by distilling a solvent off. The compound of the present disclosure or a salt thereof can also form a solvate with water or a solvent such as ethanol.

The (E)-2-(7-trifluoromethylchroman-4-ylidene)-N-(7-(-hydroxy-5,6,7,8-tetrahydronaphthalen-1-yl)acetamide has excellent antagonism against a transient receptor potential vanilloid 1 (hereinafter described as "TRPV1". The TRPV1 is also referred to as "vanilloid receptor 1 (VR1)").

The R isomer (Compound (1)), the S isomer, or the racemate of the (E)-2-(7-trifluoromethylchroman-4-ylidene)-N-(7-hydroxy-5,6,7,8-tetrahydronaphthalen-1-yl)acetamide is described in PCT International Publication No. WO2007/010383, Japanese Patent No. 4754566, Japanese Patent No. 6230743, PCT International Publication No. WO2018/221543, Japanese Patent No. 6830569, PCT International Publication No. WO2021/038889, and PCT International Publication No. WO2021/039023. The R isomer (Compound (1)), the S isomer, or the racemate can be produced by the production methods described in the above-mentioned patent documents. The contents of the above-mentioned patent documents are incorporated herein by reference in their entirety.

The TRPV1 is a TRP channel cloned from the dorsal root ganglion (DRG) as a cation channel responsive to capsaicin, is sensitive to heat of 43 °C or more and protons, and has been studied as a key nociceptive molecule (SEIKAGAKU Vol. 85, No. 7: 561-565). The TRPV1 is known to increase its activity and induce hyperalgesia upon inflammation and tissue injury. Therefore, the TRPV1 has attracted attention as a drug target candidate for use in a treatment of pain.

A TRPV1 antagonist has previously been reported to be effective in various pain models, including inflammatory pain, neuropathic pain, and osteoarthritis (SEIKAGAKU Vol. 85, No. 7: 561-565).

In the present disclosure, (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof is mixed with at least one selected from the group consisting of diquafosol, cyclosporine, hyaluronic acid, loteprednol etabonate, bromfenac, fluorometholone, and a pharmaceutically acceptable salt or solvate thereof to form an aqueous suspension liquid formulation. Thus, the (E)-2-(7-trifluoromethylchroman-4-ylidene)-N-(7-hydroxy-5,6,7,8-tetrahydronaphthalen-1-yl)acetamide or the pharmaceutically acceptable salt or solvate thereof can have improved redispersibility..

Another aspect of the present disclosure can provide a composition for improving redispersibility of an aqueous suspension liquid formulation including (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof, the composition including at least one selected from the group consisting of diquafosol, cyclosporine, hyaluronic acid, loteprednol etabonate, bromfenac, fluorometholone, and a pharmaceutically acceptable salt or solvate thereof.

Another aspect of the present disclosure can provide a composition for improving redispersibility of an aqueous suspension liquid formulation including (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof, the composition including at least one selected from the group consisting of diquafosol, cyclosporine, hyaluronic acid, loteprednol etabonate, bromfenac, fluorometholone, and a pharmaceutically acceptable salt or solvate thereof; and a nonionic surfactant.

Another aspect of the present disclosure can provide a composition for improving redispersibility of an aqueous suspension liquid formulation including (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof, the composition including at least one selected from the group consisting of diquafosol, cyclosporine, hyaluronic acid, loteprednol etabonate, bromfenac, fluorometholone, and a pharmaceutically acceptable salt or solvate thereof; a nonionic surfactant; and boric acid.

In one embodiment of the present disclosure, (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof can present in an aqueous suspension of the present disclosure at a concentration of usually about 0.01 w/v% to about 5 w/v%, preferably about 0.1 w/v% to about 3 w/v%, more preferably about 0.2 w/v% to about 2 w/v%, particularly preferably about 0.2 w/v% to about 1.5 w/v%, and further preferably about 0.3 w/v% to about 1.0 w/v%.

In one embodiment of the present disclosure, when the R isomer of (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof is used, it can present in an aqueous suspension liquid formulation of the present disclosure at a concentration of typically about 0.01 w/v% to about 5 w/v%, preferably about 0.1 w/v% to about 3 w/v%, more preferably about 0.2 w/v% to about 2 w/v%, particularly preferably about 0.2 w/v% to about 1.5 w/v%, and further preferably about 0.3 w/v% to about 1.0 w/v%.

In one embodiment of the present disclosure, the nonionic surfactant is not particularly limited as long as it is pharmaceutically acceptable. Examples thereof include tyloxapol, polyoxyl stearates (polyethylene glycol monostearate, polyethylene glycol monostearate 40 (MYS-40), polyethylene glycol monostearate 400, etc.), polyoxyethylene sorbitan fatty acid esters (polyoxyethylene sorbitan monooleate (polysorbate 80), polyoxyethylene sorbitan monolaurate, polyoxyethylene sorbitan monopalmitate, polyoxyethylene sorbitan monostearate, polyoxyethylene sorbitan tristearate, etc.), and polyoxyethylene hydrogenated castor oils (polyoxyethylene hydrogenated castor oil 10, polyoxyethylene hydrogenated castor oil 40, polyoxyethylene hydrogenated castor oil 50, polyoxyethylene hydrogenated castor oil 60 (HCO 60), etc.). The nonionic surfactant may be used alone or two or more thereof may be used in combination. The nonionic surfactant is preferably tyloxapol, polysorbate 80, polyethylene glycol monostearate, or a polyoxyethylene hydrogenated castor oil 60, more preferably tyloxapol, polysorbate 80, or polyethylene glycol monostearate, and further preferably tyloxapol from the viewpoint of redispersibility or stability of the aqueous suspension liquid formulation.

In one embodiment of the present disclosure, the nonionic surfactant can be present in the aqueous suspension liquid formulation of the present disclosure at a concentration of about 0.0001 w/v% to about 1 w/v%, preferably about 0.0005 w/v% to about 0.5 w/v%, more preferably about 0.001 w/v% to about 0.5 w/v%, particularly preferably about 0.005 w/v% to about 0.1 w/v%, and further preferably about 0.01 w/v% to about 0.05 w/v%.

In one embodiment of the present disclosure, when the tyloxapol is used as the nonionic surfactant, it can be present in the aqueous suspension liquid formulation of the present disclosure at a concentration of about 0.0001 w/v% to about 1 w/v%, preferably about 0.0005 w/v% to about 0.5 w/v%, more preferably about 0.001 w/v% to about 0.5 w/v%, particularly preferably about 0.005 w/v% to about 0.1 w/v%, and further preferably about 0.01 w/v% to about 0.05 w/v%.

In one embodiment of the present disclosure, the second component can be present in the aqueous suspension liquid formulation of the present disclosure at a concentration of about 0.005 w/v% to about 12 w/v% and preferably about 0.005 w/v% to about 10 w/v%.

In one embodiment of the present disclosure, diquafosol or a pharmaceutically acceptable salt or solvate thereof can be present in the aqueous suspension liquid formulation of the present disclosure at a concentration of about 0.3 w/v% to about 12 w/v%, preferably about 0.3 w/v% to about 10 w/v%, and more preferably about 3 w/v% to about 5 w/v%. Without wishing to be bound by any theory, the diquafosol can be mixed with (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof to thereby improve its redispersibility in the aqueous suspension liquid formulation. In this specification, the concentration of the diquafosol or the pharmaceutically acceptable salt or solvate thereof is a concentration in terms of diquafosol sodium, unless otherwise specified.

In one embodiment of the present disclosure, cyclosporine or a pharmaceutically acceptable salt or solvate thereof can be present in the aqueous suspension liquid formulation of the present disclosure at a concentration of about 0.005 w/v% to about 1 w/v%, preferably about 0.005 w/v% to about 0.5 w/v%, and more preferably about 0.05 w/v% to about 0.1 w/v%. In this specification, the concentration of the cyclosporine or the pharmaceutically acceptable salt or solvate thereof is a concentration in terms of cyclosporine (free form), unless otherwise specified.

In one embodiment of the present disclosure, hyaluronic acid or a pharmaceutically acceptable salt or solvate thereof can be present in the aqueous suspension liquid formulation of the present disclosure at a concentration of about 0.01 w/v% to about 2 w/v%, preferably about 0.01 w/v% to about 1 w/v%, and more preferably about 0.1 w/v% to about 0.5 w/v%. In this specification, the concentration of the hyaluronic acid or the pharmaceutically acceptable salt or solvate thereof is a concentration in terms of sodium hyaluronate, unless otherwise specified.

In one embodiment of the present disclosure, loteprednol etabonate or a pharmaceutically acceptable salt or solvate thereof can be present in the aqueous suspension liquid formulation of the present disclosure at a concentration of about 0.025 w/v% to about 3 w/v%, preferably about 0.025 w/v% to about 2.5 w/v%, and more preferably about 0.25 w/v% to about 1 w/v%. In this specification, the concentration of the loteprednol etabonate or the pharmaceutically acceptable salt or solvate thereof is a concentration in terms of loteprednol etabonate (free form), unless otherwise specified.

In one embodiment of the present disclosure, bromfenac or a pharmaceutically acceptable salt or solvate thereof can be present in the aqueous suspension liquid formulation of the present disclosure at a concentration of about 0.01 w/v% to about 2 w/v%, preferably about 0.01 w/v% to about 1 w/v%, and more preferably about 0.1 w/v% to about 0.5 w/v%. In this specification, the concentration of the bromfenac or the pharmaceutically acceptable salt or solvate thereof is a concentration in terms of bromfenac sodium 1.5 hydrate, unless otherwise specified.

In one embodiment of the present disclosure, fluorometholone or a pharmaceutically acceptable salt or solvate thereof can be present in the aqueous suspension liquid formulation of the present disclosure at a concentration of about 0.01 w/v% to about 2 w/v%, preferably about 0.01 w/v% to about 1 w/v%, and more preferably about 0.1 w/v% to about 0.5 w/v%. In this specification, the concentration of the fluorometholone or the pharmaceutically acceptable salt or solvate thereof is a concentration in terms of fluorometholone (free form), unless otherwise specified.

In one embodiment of the present disclosure, the aqueous suspension liquid formulation of the present disclosure may contain any additional active ingredient in addition to the first component and second component.

### (Redispersibility)

Since (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof is less soluble in water, particles thereof float on a water surface in the absence of a dispersant such as a nonionic surfactant, an ionic surfactant, or a water-soluble polymer, making it impossible to prepare an aqueous suspension liquid. In addition, if a dispersant other than a nonionic surfactant is used, it is essential to include the nonionic surfactant in the aqueous suspension liquid formulation because the dispersant needs to be included at a concentration substantially higher than a pharmaceutically acceptable concentration. The present disclosure can provide an aqueous suspension liquid formulation with improved redispersibility by including any amount of diquafosol, cyclosporine, hyaluronic acid, loteprednol etabonate, bromfenac, fluorometholone, or a pharmaceutically acceptable salt or solvate thereof in the presence of the nonionic surfactant as the dispersant.

In one embodiment of the present disclosure, an aqueous suspension liquid formulation with improved redispersibility can be provided by including at least one selected from the group consisting of diquafosol, cyclosporine, hyaluronic acid, loteprednol etabonate, bromfenac, fluorometholone, and a pharmaceutically acceptable salt or solvate thereof into (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof.

A measurement with the inversion operation can be said to reproduce evaluation when used by a patient who is weak-handed due to some disability or symptoms and has difficulty in redispersion by shaking, and thus can also be said to evaluate redispersibility when used by the patient or elderly people.

In one embodiment, the redispersibility can be determined to be improved when the number of inversion operations required for the aforementioned redispersion is typically about 44 times or less, preferably about 40 times or less, more preferably about 35 times or less, particularly preferably about 30 times or less, further preferably about 25 times or less, and most preferably about 20 times or less.

In one embodiment, the redispersibility can also be evaluated with any means, for example, by filling a container with the aqueous suspension liquid of the present disclosure and shaking the container at any rate and/or any shaking width or inverting the container to thereby measure the number of times required for suspended particles to be redispersed.

In one embodiment, an average particle size (D₅₀) of (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof in the aqueous suspension liquid of the present disclosure is not particularly limited and may be typically about 0.1 µm to about 50 µm, preferably about 0.5 µm to about 10 µm, particularly preferably about 1 µm to about 10 µm, and further preferably about 1 µm to about 5 µm. The average particle size falling within such a range can improve the redispersibility.

In one embodiment, (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof in the aqueous suspension liquid of the present disclosure can be used in a crystalline form, which is not particularly limited as long as it does not affect the redispersibility. For example, in the present disclosure, a type I crystal, a type II crystal, or a type III crystal of (E)-2-(7-trifluoromethylchroman-4-ylidene)-N-((7R)-7-hydroxy-5,6,7,8-tetrahydronaphthalene-1-yl)acetamide, or a mixture thereof disclosed in PCT International Publication No. WO 2018/221543 and Japanese Patent No. 6230743 can be used. Preferably, the type I crystal is used.

### (Dosage form)

In one embodiment of the present disclosure, the aqueous suspension liquid formulation is an ophthalmic suspension liquid formulation and can be provided as an ocular injection solution, an ophthalmic solution, or an ocular perfusate. For example, the ophthalmic suspension liquid formulation can be provided in a form of a suspension of an active ingredient in an aqueous solvent (e.g., phosphate buffered saline) or in a form of a solution of an active ingredient in an aqueous solvent.

In one embodiment of the present disclosure, the aqueous suspension liquid formulation can be an ophthalmic solution for treating a dry eye. The dry eye is a disease that is accompanied by subjective symptoms such as eye discomfort and requires a long-term and regular treatment. In addition, since a therapeutic drug for the dry eye is generally designed to be administered frequently, there is a strong need for a formulation with good patient adherence and convenience. Furthermore, there is concern that in the case of a suspension liquid with poor redispersibility, suspended particles may not be dispersed uniformly, a desired active ingredient may not be administered, and thus a pharmacological effect may not be sufficiently exerted. From these viewpoints, the redispersibility is a major issue in an ophthalmic solution for treating a dry eye, and provision of the aqueous suspension liquid formulation of the present disclosure with excellent redispersibility is highly valuable.

The aqueous suspension liquid formulation of the present disclosure can be administered by any suitable route determined by those skilled in the art and can be formulated to be suitable for administration via an administration route selected from, but not limited to, ocular injection, topical application (including application to the eye), instillation, intravenous injection, infusion, oral, parenteral, transdermal, etc.

### (Additive and/or excipient)

The aqueous suspension liquid formulation of the present disclosure may include any pharmaceutically acceptable additive and/or excipient known in the art. Examples of the additive include, but is not limited to, a viscous agent, a stabilizer, a pH adjuster, a buffer, and a preservative (antiseptic).

The viscous agent is not particularly limited to the extent that it is pharmaceutically acceptable. Examples thereof include a water-soluble polymer such as a carboxy vinyl polymer, polyvinyl pyrrolidone, polyethylene glycol, polyvinyl alcohol, xanthan gum, sodium chondroitin sulfate, sodium hyaluronate, etc.; and a cellulosic polymer such as hydroxyethyl cellulose, methyl cellulose, hydroxypropyl cellulose, hydroxypropyl methyl cellulose, sodium carboxymethyl cellulose, etc. The viscous agent may be used alone or two or more thereof may be used in combination. The cellulosic polymer is preferred and methyl cellulose is particularly preferred from the viewpoint of redispersibility.

The stabilizer is not particularly limited to the extent that it is pharmaceutically acceptable. Examples thereof include polyvinyl pyrrolidone, monoethanolamine, cyclodextrin, dextran, ascorbic acid, tocopherol, dibutylhydroxytoluene, and a sulfite. A content thereof is preferably about 0.001 w/v% to about 1 w/v% relative to a total amount of the composition.

The pH adjuster is not particularly limited to the extent that it is pharmaceutically acceptable. Examples thereof include an acid such as hydrochloric acid, acetic acid, boric acid, aminoethyl sulfonic acid, epsilon-aminocaproic acid, etc.; and an alkali such as sodium hydroxide, potassium hydroxide, borax, triethanolamine, monoethanolamine, sodium hydrogen carbonate, sodium carbonate, etc. A content thereof is, for example, 0 to about 20 w/v% relative to a total amount of the aqueous suspension liquid formulation.

In one embodiment of the present disclosure, the aqueous suspension liquid of the present disclosure may be mixed with the above-mentioned pH adjuster, if necessary, to achieve a pH of typically about 4 to about 8, preferably about 5.0 to about 8.0, more preferably about 6.0 to about 8.0, particularly preferably about 7.0 to about 8.0, and further preferably about 7.2 to about 7.8.

The buffer for use in the aqueous suspension liquid formulation of the present disclosure is not particularly limited to the extent that it is pharmaceutically acceptable. Examples thereof include a borate buffer, a phosphate buffer, a Tris buffer, a citrate buffer, a tartrate buffer, an acetate buffer, an amino acid buffer, etc., with a borate buffer or a phosphate buffer being preferred and a borate buffer being particularly preferred from the viewpoint of redispersibility or stability of the aqueous suspension liquid formulation.

A concentration of the buffer may be appropriately set within a range that can impart a desired buffering capacity to an aqueous liquid formulation and may be, for example, about 0.1 w/v% to about 10 w/v%, preferably about 1 w/v% to about 5 w/v%, and more preferably about 1 w/v% to about 3 w/v% from the viewpoint of improvement of redispersibility or stability.

The borate buffer is not particularly limited as long as it is pharmaceutically acceptable. For example, boric acid and/or a salt thereof may be used. The boric acid is not particularly limited as long as it is pharmaceutically acceptable. Examples thereof include orthoboric acid, metaboric acid, and tetraboric acid, etc. The salt of boric acid is not particularly limited as long as it is pharmaceutically acceptable. Examples thereof include a metal salt such as a sodium salt, a potassium salt, a calcium salt, a magnesium salt, an aluminum salt, etc.; and an organic amine salt such as triethylamine, triethanolamine, morpholine, piperazine, pyrrolidine, etc. The boric acid or a salt thereof may be used alone or two or more thereof may be used in combination. One suitable aspect of the borate buffer is a combination of boric acid and borax.

When boric acid and borax are used in combination, a ratio of the boric acid and the borax is not particularly limited and may be, for example, 10 to 300 parts by mass, preferably 10 to 250 parts by mass, more preferably 30 to 100 parts by mass, and particularly preferably 40 to 60 parts by mass of the borax per 100 parts by mass of the boric acid.

Specifically, the phosphate buffer may be phosphoric acid and/or a salt thereof. The salt of phosphoric acid is not particularly limited to the extent that it is pharmaceutically acceptable. Examples thereof include a dialkali metal salt of hydrogen phosphate such as disodium hydrogen phosphate, dipotassium hydrogen phosphate, etc.; an alkali metal salt of dihydrogen phosphate such as sodium dihydrogen phosphate, potassium dihydrogen phosphate, etc.; and a trialkali metal salt of phosphoric acid such as trisodium phosphate, tripotassium phosphate, etc. The salt of phosphoric acid may also be in a form of a solvate such as a hydrate, for example, disodium hydrogen phosphate may be in a form of a dodecahydrate, or sodium dihydrogen phosphate may be in a form of a dihydrate. One selected from the phosphoric acid and the salt thereof may be used alone or two or more thereof may be used in combination as the phosphate buffer. Among the phosphoric acid and the salt thereof, a phosphate is preferred, at least one of a dialkali metal salt of hydrogen phosphate and an alkali metal salt of dihydrogen phosphate is more preferred, and at least one of disodium hydrogen phosphate and sodium dihydrogen phosphate is particularly preferred.

The Tris buffer may be Tris (also known as trishydroxymethylaminomethane) and/or a salt thereof. The salt of Tris is not particularly limited as long as it is pharmaceutically acceptable. For example, a salt such as an acetate, a hydrochloride, a maleate, or a sulfonate may be used. One selected from the Tris and the salt thereof may be used alone or two or more thereof may be used in combination as the Tris buffer. In another embodiment, the Tris buffer may be specifically trometamol and/or a salt thereof. The salt of trometamol is not particularly limited to the extent that it is pharmaceutically acceptable. For example, an organic acid salt such as an acetate; an inorganic acid salt such as a hydrochloride and a sulfonate may be used. One selected from the trometamol and the salt thereof may be used alone or two or more thereof may be used in combination as the Tris buffer. Among the trometamol and the salt thereof, trometamol is preferred.

Specifically, the citrate buffer may be citric acid and/or a salt thereof. The salt of citric acid is not particularly limited to the extent that it is pharmaceutically acceptable. Examples thereof include an alkali metal salt such as a sodium salt and a potassium salt; an alkaline earth metal salt such as a calcium salt and a magnesium salt, etc. The salt of citric acid may also be in a form of a solvate such as a hydrate. One selected from the citric acid and the salt thereof may be used alone or two or more thereof may be used in combination as the citrate buffer. Among the citric acid and the salt thereof, a salt of citric acid is preferred, an alkali metal salt of citric acid is more preferred, and sodium citrate is particularly preferred.

Specifically, the tartrate buffer may be tartaric acid and/or a salt thereof. The salt of tartaric acid is not particularly limited to the extent that it is pharmaceutically acceptable. Examples thereof include an alkali metal salt such as a sodium salt and a potassium salt; an alkaline earth metal salt such as a calcium salt and a magnesium salt, etc. The salt of tartaric acid may also be in a form of a solvate such as a hydrate. One selected from the tartaric acid and the salt thereof may be used alone or two or more thereof may be used in combination as the tartrate buffer.

Specifically, the acetate buffer may be acetic acid and/or a salt thereof. The salt of acetic acid is not particularly limited to the extent that it is pharmaceutically acceptable. Examples thereof include an alkali metal salt such as a sodium salt and a potassium salt; an alkaline earth metal salt such as a calcium salt and a magnesium salt; and an ammonium salt, etc. The salt of acetic acid may also be in a form of a solvate such as a hydrate. One selected from the acetic acid and the salt thereof may be used alone or two or more thereof may be used in combination as the acetate buffer.

Specifically, the amino acid buffer may be an acidic amino acid and/or a salt thereof. Specific examples of the acidic amino acid include aspartic acid and glutamic acid. The salt of the acidic amino acid is not particularly limited to the extent that it is pharmaceutically acceptable. For example, an alkali metal salt such as a sodium salt and a potassium salt may be used. One selected from the acidic amino acid and the salt thereof may be used alone or two or more thereof may be used in combination as the amino acid buffer.

The preservative is not particularly limited as long as it is pharmaceutically acceptable. Examples thereof include sorbic acid, potassium sorbate, a paraoxybenzoate ester such as methyl paraoxybenzoate, ethyl paraoxybenzoate, propyl paraoxybenzoate, butyl paraoxybenzoate, etc., a quaternary ammonium salt such as chlorhexidine gluconate, benzalkonium chloride, benzethonium chloride, cetylpyridinium chloride, etc., alkyl polyaminoethylglycine, chlorobutanol, polyquad, polyhexamethylene biguanide, chlorhexidine, etc. A content thereof can be appropriately varied according to the type and may be, for example, about 0.0001 w/v% to about 0.2 w/v% relative to a total amount of the aqueous suspension liquid formulation.

An ophthalmic solution can be prepared, for example, by dissolving or suspending the desired components as described above in an aqueous solvent such as sterile purified water, a saline solution, a buffer solution (e.g., phosphate buffer, citrate buffer, or acetate buffer, etc.) or a non-aqueous solvent such as a vegetable oil, for example, a cottonseed oil, a soybean oil, a sesame oil, or a peanut oil, etc.; adjusting the resulting solution or suspension to a predetermined osmotic pressure; and subjecting the resultant to a sterilization treatment such as filtration sterilization.

### (Container)

A container for containing the aqueous suspension liquid formulation of the present disclosure is not particularly limited, and include, for example, a glass container or a plastic container. The plastic container can be formed of any material such as polyester (polyethylene terephthalate, polyarylate), polycarbonate, polyethylene, or polypropylene, a mixture thereof, or a mixture with another material. A container for use in the present disclosure may or may not be those used in the medical field. In one embodiment, it can be formed of any material that can meet the "Standard for Plastic Containers for Eye drops" or other equivalent standards in Japan.

The container to be used may have any shape, and any shape may typically be used as long as it is for an eye drop.

In a certain embodiment, the aqueous suspension liquid formulations of the present disclosure can be filled into any container for an eye drop commonly used in the medical field, for example, a polyethylene (preferably low-density polyethylene) container or a polypropylene container, preferably a colorless polypropylene container.

### (General tegnologies)

Molecular biological, biochemical, and microbiological procedures used herein are well known and common in the art, and are described in, for example, Sambrook J. et al. (1989). Molecular Cloning: A Laboratory Manual, Cold Spring Harbor and its 3rd Ed. (2001); Ausubel, F. M. (1987). Current Protocols in Molecular Biology, Greene Pub. Associates and Wiley-Interscience; Ausubel, F. M. (1989). Short Protocols in Molecular Biology: A Compendium of Methods from Current Protocols in Molecular Biology, Greene Pub. Associates and Wiley-Interscience; Innis, M. A. (1990). PCR Protocols: A Guide to Methods and Applications, Academic Press; Ausubel, F. M.

(1992). Short Protocols in Molecular Biology: A Compendium of Methods from Current Protocols in Molecular Biology, Greene Pub. Associates; Ausubel, F. M. (1995). Short Protocols in Molecular Biology: A Compendium of Methods from Current Protocols in Molecular Biology, Greene Pub. Associates; Innis, M. A. et al. (1995). PCR Strategies, Academic Press; Ausubel, F. M. (1999). Short Protocols in Molecular Biology: A Compendium of Methods from Current Protocols in Molecular Biology, Wiley, and annual updates; Sninsky, J.J. et al.

(1999). PCR Applications: Protocols for Functional Genomics, Academic Press, Gait, M. J. (1985). Oligonucleotide Synthesis: A Practical Approach, IRL Press; Gait, M. J. (1990). Oligonucleotide Synthesis: A Practical Approach, IRL Press; Eckstein, F. (1991). Oligonucleotides and Analogues: A Practical Approach, IRL Press; Adams, R. L. et al. (1992). The Biochemistry of the Nucleic Acids, Chapman & Hall; Shabarova, Z. et al. (1994). Advanced Organic Chemistry of Nucleic Acids, Weinheim; Blackburn, G. M. et al. (1996). Nucleic Acids in Chemistry and Biology, Oxford University Press; Hermanson, G. T. (I996). Bioconjugate Techniques, Academic Press, and Bessatsu Jikken Igaku "Experimental Methods for Gene Transfer & Expression Analysis", Yodosha, 1997, etc. These are incorporated herein by reference in its relevant parts (may be entirety).

References cited herein such as scientific literature, patents, and patent applications are incorporated herein by reference in its entirety as if specifically set forth herein.

The present disclosure has been described with reference to preferred embodiments for ease of understanding. Hereinafter, the present disclosure will be described with reference to Examples, but the above description and Examples below are provided for illustrative purposes only and are not intended to limit the present disclosure. Accordingly, the scope of the present disclosure is not limited to embodiments or Examples specifically described herein, but is limited only by claims.

### [Example]

### (Test Example 1: Evaluation of redispersibility)

### Preparation of suspension liquid

Base solutions were prepared according to compositions shown in Tables 1 to 6 and Compound (1) was added to each of the base solutions and dispersed therein with stirring. Thus, suspension liquids were obtained. Tyloxapol manufactured by AMRI Rensselaer (Curia Global, Inc.), Polysorbate 80 manufactured by NOF CORPORATION, polyethylene glycol monostearate 40 (MYS-40) manufactured by NIPPON SURFACTANT INDUSTRIES CO., LTD., and a polyoxyethylene hydrogenated castor oil 60 manufactured by NIPPON SURFACTANT INDUSTRIES CO., LTD. were used. The Compound (1) was in the form of the above-described type I crystal. Diquafosol sodium manufactured by KyongBo Pharmaceutical Co., Ltd, cyclosporine manufactured by Tokyo Chemical Industry Co., Ltd., sodium hyaluronate manufactured by SEIKAGAKU CORPORATION, roteprednol etabonate manufactured by Jinan Chenghui Shuangda Chemical Co., Ltd, bromfenac sodium manufactured by Regis, or fluorometuron manufactured by Sicor was used.

### Containerization

Each of the thus-prepared suspension liquids was collected in 5 mL portions with stirring by a stirrer and filled into eye drop containers. Each of the eye drop container was a colorless container made of polyethylene (container used for GATIFLO ophthalmic solution 0.3% (manufactured and sold by Senju Pharmaceutical Co., Ltd.)).

### Evaluation of redispersibility (inversion operation)

Suspended particles in the suspension liquid filled in the eye drop container were confirmed to have completely settled. An inversion operation (i.e., holding the container in the hand and turning it up and down) was repeated until a precipitate disappeared from a bottom surface and a wall of the eye drop container and redispersed. The number of the inversions required for redispersion was counted. The container was turned 180° vertically and then turned 180° again to stand upright at a rate of 1 second/inversion, which was defined as one inversion. The test was performed on 3 to 6 samples per formulation and an average number of inversions was calculated.

### Result

The results are shown in Tables 1 to 7. The aqueous suspension formulations each including the Compound (1) showed good redispersibility with a reduced number of inversion operations since they were formulated with diquafosol sodium, cyclosporine, sodium hyaluronate, loteprednol etabonate, bromfenac sodium, or fluorometuron, respectively. When Example 1 was evaluated by shaking operation, the number of shakes was 10.0.

**[Table 1]**

| | Component (g/100 mL) | Example 1 | Example 2 | Example 3 | Example 4 | Comparative Example 1 |
|---|---|---|---|---|---|---|
| | Compound (1) | 0.3 g | 0.3 g | 0.3 g | 0.3 g | 0.3 g |
| | Diquafosol sodium | 3.0 g | - | - | - | - |
| | Cyclosporine | - | 0.05 g | - | - | - |
| | Sodium hyaluronate | - | - | 0.1 g | - | - |
| | Loteprednol etabonate | - | - | - | 0.25 g | - |
| Formulation | Boric acid | 1.62 g | 1.62 g | 1.62 g | 1.62 g | 1.62 g |
| | Borax | 0.52 g | 0.52 g | 0.52 g | 0.52 g | 0.52 g |
| | Tyloxapol | 0.05 g | 0.05 g | 0.05 g | 0.05 g | 0.05 g |
| | Purified water | q.s. | q.s. | q.s. | q.s. | q.s. |
| | Total amount | 100 mL | 100 mL | 100 mL | 100 mL | 100 mL |
| | pH | 6.8 | 7.5 | 7.5 | 7.5 | 7.5 |
| Result | Number of inversion operation | 19.3 | 22.7 | 26.7 | 21.0 | 41.3 |
| | Percentage (% Number of inversion operation) | 46.8 | 54.8 | 64.5 | 50.8 | 100 |

**[Table 2]**

| | Component (g/100mL) | Example 5 | Example 6 | Example 7 | Example 8 | Comparative Example 2 |
|---|---|---|---|---|---|---|
| | Compound (1) | 1.0 g | 1.0 g | 1.0 g | 1.0 g | 1.0 g |
| | Diquafosol sodium | 3.0 g | - | - | - | - |
| | Cyclosporine | - | 0.05 g | - | - | - |
| | Sodium hyaluronate | - | - | 0.1 g | - | - |
| | Loteprednol etabonate | - | - | - | 0.25 g | - |
| Formulation | Boric acid | 1.62 g | 1.62 g | 1.62 g | 1.62 g | 1.62 g |
| | Borax | 0.52 g | 0.52 g | 0.52 g | 0.52 g | 0.52 g |
| | Tyloxapol | 0.05 g | 0.05 g | 0.05 g | 0.05 g | 0.05 g |
| | Purified water | q.s. | q.s. | q.s. | q.s. | q.s. |
| | Total amount | 100 mL | 100 mL | 100 mL | 100 mL | 100 mL |
| | pH | 6.7 | 7.5 | 7.5 | 7.5 | 7.5 |
| Result | Number of inversion operation | 39.5 | 43.5 | 46.0 | 37.4 | 49.5 |
| | Percentage (% Number of inversion operation) | 79.8 | 87.9 | 92.9 | 75.4 | 100 |

**[Table 3]**

| | Component (g/100mL) | Example 9 | Example 10 | Example 11 | Example 12 | Comparative Example 3 |
|---|---|---|---|---|---|---|
| | Compound (1) | 0.3 g | 0.3 g | 0.3 g | 0.3 g | 0.3 g |
| | Diquafosol sodium | 3.0 g | - | - | - | - |
| | Cyclosporine | - | 0.05 g | - | - | - |
| | Sodium hyaluronate | - | - | 0.1 g | - | - |
| | Loteprednol etabonate | - | - | - | 0.25 g | - |
| Formulation | Boric acid | 1.62 g | 1.62 g | 1.62 g | 1.62 g | 1.62 g |
| | Borax | 0.52 g | 0.52 g | 0.52 g | 0.52 g | 0.52 g |
| | Polysorbate 80 | 0.05 g | 0.05 g | 0.05 g | 0.05 g | 0.05 g |
| | Purified water | q.s. | q.s. | q.s. | q.s. | q.s. |
| | Total amount | 100 mL | 100 mL | 100 mL | 100 mL | 100 mL |
| | pH | 6.7 | 7.6 | 7.5 | 7.5 | 7.5 |
| Result | Number of inversion operation | 21.7 | 26.0 | 29.0 | 22.0 | 64.0 |
| | Percentage (% Number of inversion operation) | 33.9 | 40.6 | 45.3 | 34.4 | 100 |

**[Table 4]**

| | Component (g/100mL) | Example 13 | Comparative Example 4 |
|---|---|---|---|
| Formulation | Compound (1) | 0.3 g | 0.3 g |
| | Sodium hyaluronate | 0.1 g | - |
| | Boric acid | 1.62 g | 1.62 g |
| | Borax | 0.52 g | 0.52 g |
| | Polyethylene glycol monostearate 40 | 0.05 g | 0.05 g |
| | Purified water | q.s. | q.s. |
| | Total amount | 100 mL | 100 mL |
| | pH | 7.6 | 7.6 |
| Result | Number of inversion operation | 26.7 | 75.3 |
| | Percentage (% Number of inversion operation) | 35.4 | 100 |

**[Table 5]**

| | Component (g/100mL) | Example 14 | Comparative Example 5 |
|---|---|---|---|
| Formulation | Compound (1) | 0.3 g | 0.3 g |
| | Sodium hyaluronate | 0.1 g | - |
| | Boric acid | 1.62 g | 1.62 g |
| | Borax | 0.52 g | 0.52 g |
| | Polyoxyethylene hydrogenated castor oil 60 | 0.05 g | 0.05 g |
| | Purified water | q.s. | q.s. |
| | Total amount | 100 mL | 100 mL |
| | pH | 7.6 | 7.6 |
| Result | Number of inversion operation | 27.3 | 62.3 |
| | Percentage (% Number of inversion operation) | 43.8 | 100 |

**[Table 6]**

| | Component (g/100mL) | Example 15 | Example 16 |
|---|---|---|---|
| | Compound (1) | 0.3 g | 0.3 g |
| | Cyclosporine | 0.05 g | - |
| | Sodium hyaluronate | - | 0.1 g |
| | Boric acid | 1.62 g | 1.62 g |
| Formulation | Borax | 0.52 g | 0.52 g |
| | Tyloxapol | 0.01 g | 0.01 g |
| | Purified water | q.s. | q.s. |
| | Total amount | 100 mL | 100 mL |
| | pH | 7.5 | 7.5 |
| Result | Number of inversion operation | 23.3 | 26.0 |

**[Table 7]**

| | Component (g/100mL) | Example 17 | Example 18 | Comparative Example 1 |
|---|---|---|---|---|
| | Compound (1) | 0.3 g | 0.3 g | 0.3 g |
| | Bromfenac sodium | 0.1 g | - | - |
| | Fluorometholone | - | 0.1 g | - |
| | Boric acid | 1.62 g | 1.62 g | 1.62 g |
| Formulation | Borax | 0.52 g | 0.52 g | 0.52 g |
| | Tyloxapol | 0.05 g | 0.05 g | 0.05 g |
| | Purified water | q.s. | q.s. | q.s. |
| | Total amount | 100 mL | 100 mL | 100 mL |
| | pH | 7.5 | 7.5 | 7.5 |
| Result | Number of inversion operation | 30.3 | 25.7 | 41.3 |
| | Percentage (% Number of inversion operation) | 73.4 | 62.1 | 100 |

### (Production example)

Eye drops can be produced by preparing aqueous suspension liquid formulations having compositions shown in Table 8 to Table 9. Note that, formulated amounts of Compound (1) and additives are expressed in "g/100 mL".

**[Table 8]**

| | Ophthalmic solution No. | 1 | 2 | 3 | 4 | 5 | 6 | 7 | 8 |
|---|---|---|---|---|---|---|---|---|---|
| | Compound (1) | 0.3 | 1.0 | 0.3 | 0.3 | 0.3 | 0.3 | 0.3 | 1.0 |
| | Diquafosol sodium | 3.0 | | | | | | | |
| | Cyclosporine | | 1.0 | | | | | | |
| | Sodium hyaluronate | | | 0.1 | 0.3 | | | | |
| | Loteprednol etabonate | | | | | 0.25 | | | |
| | Bromfenac sodium | | | | | | 0.1 | | |
| | Fluorometuron | | | | | | | 0.1 | 0.02 |
| | Boric acid | 1.64 | 1.64 | 1.4 | | 1.62 | 1.62 | 1.4 | |
| | Borax | 0.54 | 0.54 | 0.35 | | 0.52 | 0.52 | 0.35 | |
| | Disodium hydrogen phosphate hydrate | | | | 0.165 | | | | 0.165 |
| | Sodium dihydrogenphosphate hydrate | | | | 0.046 | | | | 0.046 |
| Ophthalmic solution | Tyloxapol | 0.03 | 0.03 | 0.01 | 0.01 | | 0.01 | 0.05 | 0.05 |
| | Polysorbate 80 | | | | | 0.01 | | | |
| | Methylcellulose | 0.0005 | | | 0.0007 | | | | |
| | Hydroxypropyl methylcellulose | | 0.0005 | | | 0.0007 | | 0.002 | |
| | Carboxymethyl cellulose | | | 0.0005 | | | 0.0007 | | 0.002 |
| | Zinc chloride | 0.0005 | 0.0005 | | | 0.0005 | 0.0005 | | |
| | Silver nitrate | | | 0.002 | 0.002 | | | 0.003 | 0.003 |
| | Sodium chloride | | | 0.2 | 0.8 | | | 0.2 | 0.8 |
| | Sodium hydroxide | | | | q.s. | | | | q.s. |
| | Purified water | q.S. | q.s. | q.s. | q.s. | q.s. | q.s. | q.s. | q.s. |
| | Total amount | 100mL | 100mL | 100mL | 100mL | 100mL | 100mL | 100mL | 100mL |
| | pH | 7.5 | 7.5 | 7.5 | 7.5 | 7.5 | 7.5 | 7.5 | 7.5 |
| | Osmotic pressure ratio | 1 | 1 | 1 | 1 | 1 | 1 | 1 | 1 |
| | Particle size D50 | 2 to 4 | 2 to 4 | 2 to 4 | 2 to 4 | 2 to 4 | 2 to 4 | 2 to 4 | 2 to 4 |
| Container | Bottle for ophthalmic solution | Colorless PE | Colorless PP | Brown PE | Colorless PE | Colorless PP | Brown PE | Colorless PP | Colorless PE |
| | Sterilization method | EOG | EOG | EOG | EOG | EB | EB | EB | EB |

**[Table 9]**

| | Ophthalmic solution No. | 9 | 10 | 11 | 12 | 13 | 14 | 15 | 16 |
|---|---|---|---|---|---|---|---|---|---|
| | Compound (1) | 1.0 | 0.3 | 0.3 | 0.3 | 0.3 | 0.3 | 0.3 | 0.3 |
| | Rebamipide | 0.5 | | | | 2 | | | |
| | Olodaterol hydrochloride | | 0.5 | | | | 0.01 | 0.1 | |
| | Delgocitinib | | | 0.03 | 0.1 | | | | 0.3 |
| | Boric acid | 1.64 | 1.64 | 1.4 | 1.64 | 1.62 | 1.62 | 1.64 | 1.4 |
| | Borax | 0.54 | 0.54 | 0.35 | 0.54 | 0.52 | 0.52 | 0.54 | 0.35 |
| | Tyloxapol | 0.01 | 0.03 | 0.03 | 0.01 | 0.01 | 0.01 | 0.01 | 0.01 |
| | Methylcellulose | 0.003 | 0.0007 | 0.0007 | | 0.001 | 0.001 | | 0.001 |
| Ophthalmic solution | Hydroxypropyl methylcellulose | | | | 0.001 | | | 0.001 | |
| | Zinc chloride | 0.003 | | 0.003 | 0.002 | 0.002 | 0.002 | | 0.002 |
| | Sodium chloride | | | 0.2 | | | | | 0.2 |
| | Chlorhexidine gluconate | | | | | | 0.006 | 0.006 | |
| | Purified water | q.s. | q.s. | q.s. | q.s. | q.s. | q.s. | q.s. | q.s. |
| | Total amount | 100mL | 100mL | 100mL | 100mL | 100mL | 100mL | 100mL | 100mL |
| | pH | 7.5 | 7.5 | 7.5 | 7.5 | 7.5 | 7.5 | 7.5 | 7.5 |
| | Osmotic pressure ratio | 1 | 1 | 1 | 1 | 1 | 1 | 1 | 1 |
| | Particle size D50 | 2 to 4 | 2 to 4 | 2 to 4 | 2 to 4 | 2 to 4 | 2 to 4 | 2 to 4 | 2 to 4 |
| Container | Bottle for ophthalmic solution | Colorless PE | Colorless PP | Brown PE | Colorless PE | Colorless PP | Brown PE | Colorless PP | Colorless PE |
| | Sterilization method | EOG | EOG | EOG | EB | EB | EB | Gamma ray | VHP |

Tyloxapol (clouding point: 90 to 100 °C) manufactured by AMRI Rensselaer (Curia Global, Inc.), METOLOSE SM-15 manufactured by Shin-Etsu Chemical Co., Ltd. serving as methylcellulose, 60SH-4000 made by Shin-Etsu Chemical Co., Ltd. serving as hydroxypropyl methylcellulose (HPMC), CELLOGEN PR-S manufactured by DKS Co. Ltd. serving as carboxymethylcellulose (CMC), and lifitegrast manufactured by Shanghai Sumway Pharmaceutical Technology are used. The Compound (1) is in the form of the type I crystal.

### Specification of container

Containers are constituted of a bottle (colorless PE, colorless PP, or brown PP), a nozzle (PE) and a cap (PP). Appearance, lightness, chromaticity, saturation, and transmittance at various wavelengths of a body of the bottle of each of the containers are as per specifications shown in Table 10 and FIG. 1. Each of the containers is also shrink-wrapped for a product label.

**[Table 10]**

| | | | Lightness | Chromaticity | | | |
|---|---|---|---|---|---|---|---|
| Bottle | Material | Appearance | *L** | *a** | *b** | Saturation | Transmittance |
| Colorless PE | Polyethylene | Colorless (white) | 69.39 | 1.54 | 13.83 | 13.92 | FIG. 1 |
| Colorless PP | Polypropylene | Colorless (white) | 90.90 | 0.07 | 5.20 | 5.20 | FIG. 1 |
| Brown PE | Polyethylene | Brown | 67.33 | 1.15 | 27.57 | 27.59 | FIG. 1 |

### Shape of bottle

Each of the bottles has a dimension of about 23 m x about 17 mm x about 50 mm and a resin weight of approximately 3 g. A shape thereof is similar to that of a SOFTEAR (registered trademark) ophthalmic solution 0.02% (manufactured and distributed by Senju Pharmaceutical CO., LTD.).

### Material of bottle

Polyethylene denotes low-density polyethylene. Polypropylene denotes a propylene-ethylene copolymer composed of a propylene component/ethylene component = 50/50 to 99.9/0.1, and is the same as a material of the bottle of the SOFTEAR (registered trademark) ophthalmic solution 0.02% (manufactured and distributed by Senju Pharmaceutical CO., LTD.).

### Method for measuring lightness, saturation, and transmittance of body of bottle

A side section (wider side section, 1.0 cm x 2.0 cm to 3.0 cm) cut from the body of the bottle is measured for the lightness (L* value) and the chromaticity (a* value, b* value) using a spectrophotometer (CM-5, manufactured by KONICA MINOLTA, INC.). The saturation (c* value) was also calculated according to the following expression: ((a* value)² + (b* value)²)^{1/2}. The transmittance (%) of light in a region of 200 to 800 nm is measured for the side section using a UV-visible spectrophotometer ("Model UV-2450," manufactured by SHIMADZU CORPORATION) .

### Sterilization method of bottle

EB (electron beam sterilization): A bottle for an eye drop is sterilized by irradiation with electron beams at 10 to 60 kGy.

EOG (ethylene oxide gas sterilization): A bottle for an eye drop is sterilized under the following conditions: an ethylene oxide concentration of 400 to 700 mg/L, a temperature of 40 to 50 °C, a relative humidity of 45 to 85%, and a treatment time of 3 hours or more.

VHP (vaporous hydrogen peroxide sterilization): A bottle for an eye drop is sterilized under the following conditions: spraying of 3% VHP, a temperature of 20 to 50 °C, a relative humidity of 30 to 90%, and a treatment time of about 1 hour.

Gamma ray (γ-ray sterilization): A bottle for an eye drop is sterilized by irradiation with gamma rays at 20 to 60 kGy.

### Method of producing ophthalmic solution at 50L scale

Purified water and predetermined amounts of boric acid, borax, sodium chloride, and zinc chloride are added to a 30 L stainless steel container and stirred. A predetermined amount of an aqueous tyloxapol solution of is prepared in a 1 L stainless steel container and added to the 30 L stainless steel container. In another 1 L stainless steel vessel, a predetermined amount of methylcellulose is dispersed in hot water (50 to 90 °C), cooled, and the resulting aqueous methylcellulose solution is added to the 30 L stainless steel container. Once all additives have been confirmed to be dissolved in the 30 L stainless steel container, purified water is added thereto to make a dilution by weight to a 1.5 X base solution. The base solution is sterilized by filtration, and diluted by weight in purified water (including for washing of insoluble residues) to prepare a predetermined amount of a base solution. The Compound (1) serving as the first component and the second component (diquafosol sodium, cyclosporine, sodium hyaluronate, loteprednol etabonate, bromfenac sodium, fluorometuron, rebamipide, olodaterol hydrochloride, or dercositinib) are added to the base solution and dispersed with stirring to prepare a suspension liquid. After confirming that the Compound (1) is dispersed in the liquid, the suspension liquid was gently stirred for defoaming. The suspension liquid is then coarsely filtered through a filter with a pore diameter of 75 µm, the thus-filtered suspension liquid is added to a bottle with stirring, and then a nozzle and a cap are installed to the bottle.

### (Notes)

Although the present disclosure has been illustrated with reference to preferred embodiments of the present disclosure, it is understood that the scope of the present disclosure should be construed only by the claims. It is to be understood that the patents, patent applications, and other references cited herein should be incorporated herein by reference in its entirety as if the contents themselves are specifically set forth herein. This application claims priority to Japanese Patent Application No. 2022-156062 filed with the Japan Patent Office, the content of which is incorporated herein by reference in their entirety.

### [Industrial Applicability]

The present disclosure is available in the fields of medicine, pharmaceuticals, healthcare, biology, biochemistry, etc.

## Claims

1. An aqueous suspension liquid formulation comprising:
a first component; and
a second component,
the first component being (E)-2-(7-trifluoromethylchroman-4-ylidene)-N-(7-hydroxy-5,6,7,8-tetrahydronaphthalen-1-yl)acetamide or a pharmaceutically acceptable salt or solvate thereof, and
the second component being at least one selected from the group consisting of diquafosol, cyclosporine, hyaluronic acid, loteprednol etabonate, bromfenac, fluorometholone, and a pharmaceutically acceptable salt or solvate thereof.

2. The aqueous suspension liquid formulation according to claim 1, further comprising a nonionic surfactant.

3. The aqueous suspension liquid formulation according to claim 2, wherein the nonionic surfactant is at least one selected from the group consisting of tyloxapol, polysorbate 80, polyethylene glycol monostearate 40, and a polyoxyethylene hydrogenated castor oil 60.

4. The aqueous suspension liquid formulation according to claim 2, wherein the nonionic surfactant is tyloxapol.

5. The aqueous suspension liquid formulation according to claim 2, wherein the nonionic surfactant has a concentration of about 0.0001 w/v% to about 1 w/v% in the aqueous suspension liquid formulation.

6. The aqueous suspension liquid formulation according to claim 1, wherein the first component has a concentration of about 0.01 w/v% to about 5 w/v% in the aqueous suspension liquid formulation.

7. The aqueous suspension liquid formulation according to claim 1, wherein the diquafosol or a pharmaceutically acceptable salt or solvate thereof has a concentration of about 0.3 w/v% to about 10 w/v% in the aqueous suspension liquid formulation;
the cyclosporine or a pharmaceutically acceptable salt or solvate thereof has a concentration of about 0.005 w/v% to about 0.5 w/v% in the aqueous suspension liquid formulation;
the hyaluronic acid or a pharmaceutically acceptable salt or solvate thereof has a concentration of about 0.01 w/v% to about 1 w/v% in the aqueous suspension liquid formulation;
the loteprednol etabonate or a pharmaceutically acceptable salt or solvate thereof has a concentration of about 0.025 w/v% to about 2.5 w/v% in the aqueous suspension liquid formulation;
the bromfenac or a pharmaceutically acceptable salt or solvate thereof has a concentration of about 0.01 w/v% to about 1 w/v% in the aqueous suspension liquid formulation; and
the fluorometholone or a pharmaceutically acceptable salt or solvate thereof has a concentration of about 0.01 w/v% to about 1 w/v% in the aqueous suspension liquid formulation.

8. The aqueous suspension liquid formulation according to any one of claims 1 to 7, wherein the first component is (E)-2-(7-trifluoromethylchroman-4-ylidene)-N-((7R)-7-hydroxy-5,6,7,8-tetrahydronaphthalen-1-yl)acetamide or a pharmaceutically acceptable salt or solvate thereof.
